(19) Europäisches Patentamt
European Patent Office
Office européen des brevets

(11) Publication number: **0 487 155 A1**

(12) **EUROPEAN PATENT APPLICATION**

(21) Application number: **91202988.1**

(51) Int. Cl.5: **C07D 243/12**, A01N 43/62

(22) Date of filing: **18.11.91**

(30) Priority: **23.11.90 US 617241**

(43) Date of publication of application: **27.05.92 Bulletin 92/22**

(84) Designated Contracting States: **DE FR GB**

(71) Applicant: **EASTMAN KODAK COMPANY**
**343 State Street**
**Rochester, New York 14650-2201(US)**

(72) Inventor: **Delany, John James, c/o EASTMAN KODAK COMPANY**
**Patent Department, 343 State Street**
**Rochester, New York 14650(US)**
Inventor: **Cook, Ewell Russell, c/o EASTMAN KODAK COMPANY**
**Patent Department, 343 State Street**
**Rochester, New York 14650(US)**

(74) Representative: **Nunney, Ronald Frederick Adolphe et al**
**Kodak Limited Patent Department Headstone Drive**
**Harrow Middlesex HA1 4TY(GB)**

(54) **Substituted-2,3-dihydro-2-oxo-1,5-benzodiazepines and their use as fungicides.**

(57) A method of controlling fungi comprising contacting the fungi with a fungicidally effective amount of a benzodiazepine of the structure:

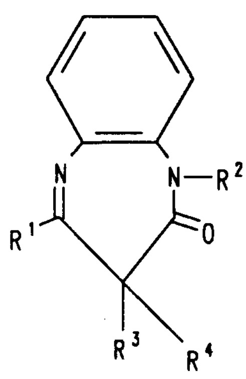

wherein

$R^1$ is substituted or unsubstituted, straight or branched alkyl having from 3 to 10 carbon atoms, or substituted or unsubstituted aryl having from 6 to 20 carbon atoms,

$R^2$ is substituted or unsubstituted, straight or branched alkyl having from 1 to 10 carbon atoms, or substituted or unsubstituted aryl having from 6 to 20 carbon atoms,

$R^3$ is hydrogen, halogen, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen,

provided that when R4 is halogen, $R^3$ is the same halogen.

EP 0 487 155 A1

The present invention relates to the fungicidal use of substituted 2,3-dihydro-2-oxo-1,5-benzodiazepines.

In the food agricultural industry, there is a continuing need to provide the public with a variety of fungicidal methods.

The compounds useful in the fungicidal method of this invention are substituted-2,3-dihydro-2-oxo-1,5-benzodiazepines.

The synthesis of certain 1,5-benzodiazepines and alkylation of the 1-position nitrogen of the ring has been reported.

"Herbicidal and Pesticidal Properties of Some 1,5-Benzodiazepines, 1,3,5-Benzotriazepines and 3,1,5-Benzothiadiazepines" by D. Clifford, D. Jackson, R. Edwards and P. Jeffrey, Pest. Sci., Vol. 7, pages 453-458, (1976), discloses the preparation of certain benzodiazepines having the structure:

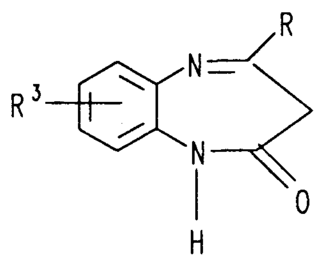

wherein R is methyl or hydrogen. It also discloses the investigation of biological activity of the benzodiazepines, including herbicidal, insecticidal, and fungicidal activity.

The need to discover new, alternative methods of controlling fungi continues. More variety is needed. Fungi remain a problem in food agriculture.

The problem noted above has been solved upon our discovery of a method of controlling fungi comprising contacting the fungi with a fungicidally effective amount of a benzodiazepine,

the benzodiazepine characterized by the following structure:

I

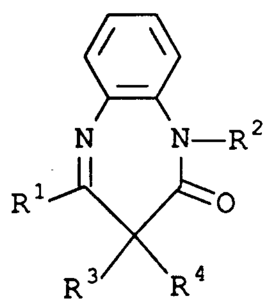

wherein

$R^1$ is substituted or unsubstituted alkyl having from 3 to 10 carbon atoms, or substituted or unsubstituted aryl having from 6 to 20 carbon atoms,

$R^2$ is substituted or unsubstituted alkyl having from 1 to 10 carbon atoms, or substituted or unsubstituted aryl having from 6 to 20 carbon atoms,

$R^3$ is hydrogen, halogen, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen, provided that when $R^4$ is halogen, $R^3$ is the same halogen.

Also provided are novel substituted 1,5- benzodiazepine compounds,

the compounds characterized as defined by the structure above and additionally with the following provisions:

when $R^2$ is a halogen, the halogen is bromo, chloro or iodo; and when $R^1$ is methyl or phenyl and $R^2$ is allyl, ethyl, benzyl, propyl or butyl, then neither $R^3$ nor $R^4$ are hydrogen.

Further, a fungicidal composition is provided comprising a fungicidally effective amount of a benzodiazepine,

the benzodiazepine characterized by the structure I, defined above.

Still further, the invention provides a new use of substituted 1,5-benzodiazepine derivatives as fungicides.

It is an advantageous feature of the invention that it provides an alternative to known fungicidal methods.

It is also an advantageous feature of the invention that fungicidal method provides 80 to 100 percent control against at least one fungus.

More particularly, the benzodiazepine compounds are of the stucture I shown above wherein:

$R^1$ is substituted or unsubstituted, straight or branched alkyl having from 3 to 10 carbon atoms, for example, propyl, isopropyl, butyl, isobutyl, tertiary butyl, pentyl, benzyl, hexyl, octyl, nonyl, isononyl, decyl, hydroxypropyl, dihydroxypropyl, chlorobutyl and bromooctyl, or

substituted or unsubstituted aryl having from 6 to 20 carbon atoms, for example, phenyl, 4-chlorophenyl, tolyl, xylyl, 2,4-dichlorophenyl, 2,4,6-trichlorophenyl, 2,4,6-tribromophenyl, 1-naphthyl and 2-naphthyl.

$R^2$ is substituted or unsubstituted, straight or branched alkyl having from 1 to 10 carbon atoms, such as the alkyl groups described previously for $R^1$, and also including perfluoroethyl, phenethyl, trifluoromethyl, methyl, and ethyl, and

substituted or unsubstituted aryl having from 6 to 20 carbon atoms, such as the aryl described for $R^1$.

$R^3$ is hydrogen, halogen, such as chloro, iodo, fluoro and bromo, substituted and unsubstituted alkyl having from 1 to 10 carbon atoms, such as the examples described previously for $R^1$, alkenyl having from 2 to 10 carbon atoms such as propenyl, butenyl, pentenyl, hexenyl, octenyl and decenyl, or substituted and unsubstituted aryl having from 6 to 20 carbon atoms, such as the examples described previously for $R^1$.

$R^4$ is hydrogen or halogen, such as chloro, iodo, fluoro, and bromo, provided that when $R^4$ is halogen, $R^3$ is the same halogen.

Preferably the 1,5-benzodiazepines are defined wherein: $R^1$ is an alkyl of 3 to 6 carbon atoms or a substituted or unsubstituted aryl of 6 to 10 carbon atoms, $R^2$ is an alkyl of 1 to 8 carbon atoms, and $R^3$ is an alkyl of 1 to 6 carbon atoms, an alkenyl of 2 to 4 carbon atoms, or a bromo. More preferably, $R^1$ is a tertiary butyl, phenyl, or 4-chlorophenyl, $R^2$ is methyl, ethyl, n-propyl, n-butyl or benzyl, and $R^3$ is hydrogen, allyl, bromo, chloro, n-butyl, ethyl, or methyl. Most preferably, $R^1$ is phenyl, $R^2$ is methyl and $R^3$ is ethyl; $R^1$ is phenyl, $R^2$ is methyl and $R^3$ is n-butyl; or $R^1$ is chlorophenyl, $R^2$ is methyl and $R^3$ is bromo.

Compounds representative of those useful as fungicides include: 1-n-butyl-2,3-dihydro-2-oxo-4-phenyl-1H-1,5-benzodiazepine; 1-benzyl-4-t-butyl-2,3-dihydro-2-oxo-1H-1,5-benzodiazepine; 3-bromo-2,3-dihydro-1-methyl-2-oxo-4-phenyl-1H-1,5-benzodiazepine; 3-ethyl-2,3-dihydro-1-methyl-2-oxo-4-phenyl-1H-1,5-benzodiazepine; 3-n-butyl-2,3-dihydro-1-methyl-2-oxo-4-phenyl-1H-1,5-benzodiazepine; 4-t-butyl-2,3-dihydro-3-ethyl-1-methyl-2-oxo-1H-1,5-benzodiazepine; 2,3-dihydro-1,3-dimethyl-2-oxo-4-phenyl-1H-1,5-benzodiazepine; 2,3-dihydro-1-ethyl-2-oxo-4-phenyl-1H-1,5-benzodiazepine; 2,3-dihydro-2-oxo-4-phenyl-1-propyl-1H-1,5-benzodiazepine; 2,3-dihydro-1-ethyl-3-methyl-2-oxo-4-phenyl-1H-1,5-benzodiazepine; 1,3-diethyl-2,3-dihydro-2-oxo-4-phenyl-1H-1,5-benzodiazepine; 2,3-dihydro-3-methyl-2-oxo-4-phenyl-1-n-propyl-1H-1,5-benzodiazepine; 4-(4-chlorophenyl)-2,3-dihydro-1,3-dimethyl-2-oxo-1H-1,5-benzodiazepine; and 4-(4-chlorophenyl)-2,3-dihydro-3-ethyl-1-methyl-2-oxo-1H-1,5-benzo-diazepine.

By employing a fungicidally effective amount of the substituted 1,5-benzodiazepine in the invention, it is meant that the concentration of the compound allows for control of the fungi. For example, when the compound is applied in solution, it should be present in a concentration from 1% to 90% of the entire composition. When applied in an emulsifiable concentration, the compound should be present in an amount from 10% to 90% of the entire composition. When applied in a dust, the compound is generally present in an amount from 20% to 80% then subsequently diluted to 1% to 10% when wetted for actual use.

The present invention provides a means for controlling a number of types of fungi. For example, in testing the inventive composition, enhanced activity on at least one of the following fungi has been found: Colletotrichum graminicola (Anthracnose on corn), Alternaria solani (Early blight on tomatoes), Botrytis cinerea (gray mold on peppers), Phytophthora infestans (late blight on tomatoes), Erysiphe graminis f.sp. tritici (Powdery mildew on wheat), and Puccinia recondita f. sp. tritici (Wheat leaf rust).

The 1,5-benzodiazepine derivatives are generally obtainable as colorless to yellow oils having characteristic absorption spectra and which may be purified by column chromatography using common organic solvents. They are appreciably soluble in many organic solvents such as methanol, ethanol, acetone, chloroform, benzene, dioxane, dimethyl sulfoxide and N,N-dimethylformamide, but are relatively insoluble in water.

The 1,5-benzodiazepines of this invention can generally be prepared by known methods involving the condensation of an o-phenylenediamine with an alkyl 3-oxoalkanoate, for example, in boiling xylene.

The reaction scheme is generally illustrated as follows:

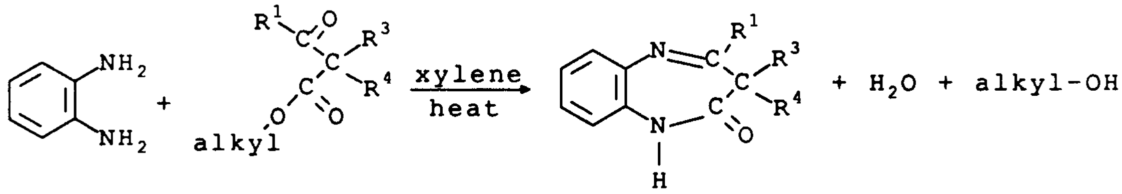


The 1-position can then be alkylated and subsequently, the 3 and 4-positions of the diazepine, if not substituted, can then be alkylated with conventional alkylating agents such as methyl iodide, ethyl iodide, benzyl bromide, and so forth, in the presence of potassium t-butoxide, as illustrated in Examples II to V of this application.

The 1,5-benzodiazepines useful in the invention can be applied as fungicidal sprays by methods commonly employed at varying concentrations, air-blast, aerial sprays and dusts. The dilution and rate of application will depend upon the type of equipment employed, the method and frequency of application desired and the type of fungi to be controlled.

Such active compounds may be employed alone or in the form of fungicidal compositions where the active ingredient is employed in a fungicidally effective amount and combined with solid and/or liquid carrier vehicles and/or with other known compatible active agents, especially plant protection agents, such as other insecticides, nematocides, or acaricides, fungicides, bactericides, rodenticides, herbicides, fertilizers, growth-regulating agents, and so forth, if desired, or in the form of particular dosage preparations for specific application made therefrom, such as solutions, emulsions, suspensions, powders, pastes, and granules which are thus ready for use.

The process of the present invention is useful for the control of various fungi as described herein, and can be utilized on the foliage. For such purposes, these compounds can be used in solutions, liquid formulations, or dry powder formulations. The compounds are usually taken up in a carrier or are formulated so as to render them suitable for subsequent use as fungicides. For example, these chemical agents can be formulated as wettable powders, dry powders, emulsifiable concentrates, dusts, granular formulations, aerosols, or flowable emulsion concentrates. In such formulations, the compounds are extended with a liquid or solid carrier and suitable surfactants are incorporated.

It is usually desirable, particularly in the case of foliar spray formulations, to include adjuvants, such as wetting agents, spreading agents, dispersing agents, stickers, adhesives and the like in accordance with agricultural practices.

In general, the compounds utilized in this invention can be dissolved in appropriate solvents such as acetone, methanol, ethanol, dimethylformamide or methyl sulfoxide and such solutions extended with water. The concentration of the solution can vary from 1 to 90% with a preferred range being 5 to 50%.

For the preparation of emulsifiable concentrates, the compounds useful in the invention can be dissolved in suitable organic solvents or a mixture of solvents, together with an emulsifying agent which permits dispersion of the fungicide in water. The concentration of the active ingredient in emulsifiable concentrates is usually 10 to 90% and in flowable emulsion concentrates, this can be as high as 75%.

Wettable powders suitable for spraying, can be prepared by admixing the compound with a finely divided solid, such as clays, inorganic silicates and carbonates, and silicas and incorporating wetting agents, sticking agents, and/or dispersing agents in such mixtures. The concentration of active ingredients in such formulations may vary widely.

Dusts can be prepared by mixing the substituted 2,3-dihydro-2-oxo-1,5-benzodiazepines with finely divided inert solids which can be organic or inorganic in nature. Materials useful for this purpose include botanical flours, silicas, silicates, carbonates and clays. One convenient method of preparing a dust is to dilute a wettable powder with a finely divided carrier. Dust concentrations containing 20% to 80% of the active ingredient are commonly made and are subsequently diluted to 1% to 10% use concentration.

## Example I

The compounds useful in this invention were evaluated as protectant fungicides by a standard method as follows. The results of these primary evaluations are recorded in Table II. Furthermore, these compounds were evaluated for their dose response profiles against mildew as shown in Table III.

### Plant Disease Control Assay

Primary Screening for Fungicidal Activity (Protectant)

Tomatoes, peppers, beans and corn were germinated and grown for one to three weeks (depending on species) in the greenhouse. Two pots representing two replicates of each plant species were placed in a flat such that each flat contained all the plants that were sprayed by one compound. The plants in each flat were sprayed to runoff at the rate of 135 parts per million active ingredient with either a test compound or fungicide standard. As a control, check plants were sprayed with water. The plants were allowed to air dry two to three hours. After drying, the plants were sorted and grouped by plant species.

Plant pathogenic fungi (Phytophthora infestans, Alternaria solani, Botrytis cinerea, Colletotrichum graminicola) were grown in the laboratory on appropriate media. Inoculum from each fungus was harvested and concentrations adjusted to predetermined levels. The obligate plant pathogenic fungi (Erysiphe graminis f.sp. tritici, Puccinia recondita f.sp. tritici) were harvested from their hosts in the greenhouse and concentrations were adjusted to predetermined levels.

The plants previously treated with test compounds were sprayed with fungal inoculum and then placed in humidity chambers for a period of time previously determined to be optimum for development of each disease. After incubation, the plants were moved to the greenhouse, symptoms allowed to develop (one week), and the plants were evaluated for disease intensity. The reported percent disease control represents the average of the two replicates.

Secondary Screening for Fungicidal Activity

(Protectant, Systemic)

Compounds providing a minimum of 75% disease control for at least two diseases or compounds demonstrating efficacy greater than 80% for a single disease were automatically advanced to secondary screening. The results of the secondary screening are recorded in Table III.

Fungicidal efficacy was titrated to the no effect level on the diseases controlled in the primary screen. Plants were grown and prepared for spraying as in the primary screen except that three replicates were used. The test compounds or fungicide standards were sprayed to runoff at concentrations of 5, 15, 45 and 135 parts per million. As a control, check plants were sprayed with water. The plants were allowed to air-dry and then fungal inoculum was applied as in the primary screen.

The inoculated plants from the protectant spray studies were incubated in humidity chambers, moved to the greenhouse for symptom development, then the concentration required to provide 50% disease control was calculated by comparison of treated and untreated plants, the results being expressed in parts per million.

The reported concentrations are the averages of three replicates.

Example II

1-Methyl-4-phenyl,-2,3-dihydro-2-oxo-1,5-benzodiazepine

To 4.0 g of 4-phenyl-2,3-dihydro-2-oxo-1,5-benzodiazepine in 50 ml anhydrous tetrahydrofuran (THF) was added 2.1 g powdered potassium t-butoxide. The resulting mixture was stirred at room temperature for 10 min. Methyl iodide (1.25 ml) was added and the resulting colorless solution was stirred at room temperature for 45 min. The mixture was diluted with saturated ammonium chloride solution (50 ml) and extracted three times with ethyl acetate (50 ml). The organic extracts were dried ($MgSO_4$), filtered, and evaporated. Chromatography of the resulting oil on silica gel gave 4.2 g (99%) of 1-methyl-4-phenyl-2,3-dihydro-2-oxo-1,5-benzodiazepine as a light yellow oil. $^1$H NMR spectrum: ($CDCl_3$d) 8.15 (2H, m), 7.45 (4H, m), 7.25 (3H, m), 3.80 (1H, d, J = 12 Hz), 3.35 (3H, s), 2.97 (1H, d, J = 12 Hz).

Example III

1,3-Dimethyl-4-phenyl-2,3-dihydro-2-oxo-1,5-benzodiazepine.

To 1.07 g of 1-methyl-4-phenyl-2,3-dihydro- 2-oxo-1,5-benzodiazepine in 30 ml anhydrous tetrahydrofuran was added 0.62 g powdered potassium t-butoxide. The resulting dark red mixture was stirred at room temperature for 5 min. Methyl iodide (0.32 ml) was added and the resulting solution was stirred at room temperature for 45 min. During this period, the reaction mixture turned from red to light yellow and a

white solid precipitate formed. The mixture was diluted with saturated ammonium chloride solution (50 ml) and extracted three times with methylene chloride (50 ml). The organic extracts were dried ($MgSO_4$), filtered and evaporated. Chromatography of the resulting oil on silica gel gave 0.74 g (65%) of 1,3-dimethyl-4-phenyl-2,3-dihydro-2-oxo-1,5-benzodiazepine as a light yellow oil. $^1$H NMR spectrum: ($CDCl_3$d) 7.60 (2H, m), 7.35 (7H, m), 3.45 (3H, s), 3.10 (1H, d, J = 7 Hz), 1.40 (3H, d, J = 7 Hz).

Example IV

3-Ethyl-1-methyl-4-phenyl-2,3-dihydro-2-oxo-1,5-benzodiazepine

To 0.38 g of 1-methyl-4-phenyl-2,3-dihydro-2-oxo-1,5-benzodiazepinein 10 ml anhydrous THF was added 0.17 g powdered potassium t-butoxide. The resulting dark red mixture was stirred at room temperature for 5 min. Ethyl iodide (0.17 ml) was added and the resulting solution was stirred at room temperature for 45 min. During this period, the reaction mixture turned from red to light yellow and a white solid precipitate formed. The mixture was diluted with saturated ammonium chloride solution (50 ml) and extracted three times with methylene chloride (50 ml). The organic extracts were dried ($MgSO_4$), filtered, and evaporated. Chromatography of the resulting oil on silica gel gave 0.31 g (73%) of 3-ethyl-1-methyl-4-phenyl-2,3-dihydro-2-oxo-1,5-benzodiazepine as light yellow oil. $^1$H NMR spectrum: ($CDCl_3$d) 7.60 (2H, m), 7.35 (7H, m), 3.45 (3H, s), 2.85 (1H, m), 2.30 (1H, m), 1.80 (1H, m) 0.75 (3H, d, J = 7 Hz).

Example V

1-Benzyl-4-t-butyl-2,3-dihydro-2-oxo-1,5-benzodiazepine

To 1.6 g of 4-t-butyl-2,3-dihydro-2-oxo-1,5-benzodiazepine in 50 ml anhydrous THF was added 0.83 g powdered potassium t-butoxide. The resulting mixture was stirred at room temperature for 10 min. Benzyl bromide (0.97 ml) was added and the resulting colorless solution was stirred at room temperature for 45 min. The mixture was diluted with saturated ammonium chloride solution (50 ml) and extracted three times with ethyl acetate (50 ml). The organic extracts were dried ($MgSO_4$), filtered and evaporated. Chromatography of the resulting oil on silia gel gave 1.85 g (82%) of 1-benzyl-4-t-butyl-2,3-dihydro-2-oxo-1,5-benzodiazepine as a light yellow oil. $^1$H NMR spectrum: ($CDCl_3$d) 7.45 (4H, m), 7.25 (5H, m), 3.80 (1H, d, J = 12 Hz), 3.5 (2H, s), 2.80 (1H, d, J = 12 Hz), 1.30 (9H, s).

Table I

| Pathogens | | | |
|---|---|---|---|
| Common Name | Scientific Name | Host | Abbr. |
| Anthracnose | Colletotrichum graminicola | Corn | MA |
| Early blight | Alternaria solani | Tomato | EB |
| Gray Mold | Botrytis cinerea | Pepper | BC |
| Late blight | Phytothora infestans | Tomato | LB |
| Powdery mildew | Erysiphi gramanis f.sp. tritici | Wheat | WM |
| Rust | Puccinia recondita f.sp. tritici | Wheat | WR |

Table II

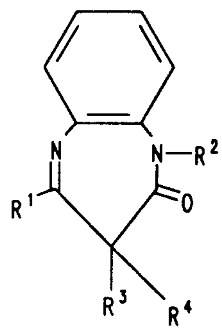

| CONTROL* | $R^1$ | $R^2$ | $R^3$ | LB[a.] | EB[b.] | BC[c.] | MA[d.] | WR[e.] | WM[f.] |
|---|---|---|---|---|---|---|---|---|---|
| 1 | Me | H | H | 0 | 0 | 0 | 0 | 0 | 1 |

| COMPOUND* | $R^1$ | $R^2$ | $R^3$ | LB | EB | BC | MA | WR | WM |
|---|---|---|---|---|---|---|---|---|---|
| 1 | t-Bu | H | H | 0 | 0 | 0 | 0 | 0 | 2 |
| 2 | t-Bu | H | Cl | 0 | 0 | 0 | 0 | 0 | 2 |
| 3 | t-Bu | Me | H | 0 | 1 | 0 | 0 | 0 | 0 |
| 4 | Ph | Me | H | 0 | 0 | 0 | 0 | 0 | 3 |
| 5* | Ph | Me | Cl | 0 | 0 | 0 | 0 | 0 | 2 |
| 6 | Ph | Me | Cl | 0 | 0 | 0 | 0 | 0 | 1 |
| 7 | t-Bu | n-Bu | H | 0 | 0 | 0 | 0 | 0 | 0 |
| 8 | Ph | n-Bu | H | 0 | 2 | 0 | 0 | 0 | 4 |
| 9 | t-Bu | Bz | H | 0 | 0 | 0 | 0 | 0 | 4 |
| 10 | Ph | Bz | H | 0 | 0 | 3 | 0 | 0 | 0 |
| 11 | Ph | Me | allyl | 1 | 0 | 0 | 0 | 0 | 3 |
| 12 | Ph | Me | Br | 0 | 0 | 0 | 0 | 1 | 3 |
| 13 | Me | Et | H | 3 | 0 | 0 | 0 | 0 | 2 |
| 14 | t-Bu | Et | H | 0 | 0 | 0 | 0 | 0 | 3 |
| 15 | Ph | Me | Et | - | 0 | 0 | 0 | 0 | 4 |
| 16 | Ph | Me | n-Bu | - | 0 | 0 | 1 | 0 | 4 |
| 17 | Ph | Me | Bz | - | 0 | 3 | 0 | 0 | 0 |

*$R^4$ is Cl in Example 5 and is H in all other examples.
a.-f. are defined in Table I.

| COMPOUND | $R^1$ | $R^2$ | $R^3$ | LB | EB | BC | MA | WR | WM |
|---|---|---|---|---|---|---|---|---|---|
| 18 | t-Bu | Me | Et | - | 0 | 0 | 0 | 0 | 4 |
| 19 | t-Bu | Me | n-Bu | - | 3 | 0 | 0 | 0 | 0 |
| 20 | t-Bu | Me | allyl | - | 3 | 0 | 0 | 0 | 0 |
| 21 | Ph | Me | Me | 0 | 0 | 0 | 0 | 0 | 4 |
| 22 | Ph | Et | H | 0 | 0 | 0 | 0 | 0 | 4 |
| 23 | Ph | n-propyl | H | 0 | 0 | 0 | 0 | 0 | 3 |
| 24 | Ph | Et | Me | 0 | 0 | 0 | 0 | 0 | 3 |
| 25 | Ph | Et | Et | 0 | 0 | 0 | 0 | 0 | 4 |
| 26 | Ph | n-propyl | Me | 0 | 0 | 0 | 0 | 0 | 4 |
| 27 | 4-ClPh | Me | Me | 0 | 0 | 0 | 0 | 0 | 3 |
| 28 | 4-ClPh | Me | Et | 0 | 0 | 0 | 0 | 0 | 3 |
| 29 | t-Bu | Et | Cl | - | 0 | 0 | 0 | 0 | 0 |
| 30 | t-Bu | Me | Bz | - | 0 | 0 | 0 | 0 | 0 |
| 31 | Me | allyl | H | - | 0 | 0 | 0 | 0 | 0 |
| 32 | Me | n-propyl | H | 0 | 0 | 0 | 0 | 0 | 0 |
| 33 | Ph | Me | n-propyl | 0 | 0 | 0 | 0 | 0 | 0 |
| 34 | Ph | n-propyl | Et | 0 | 0 | 0 | 0 | 0 | 0 |
| 35 | p-MePh | Me | Me | 0 | 0 | 0 | 0 | 0 | 0 |
| 36 | p-MePh | Me | Et | 0 | 0 | 0 | 0 | 0 | 0 |
| 37 | 3,4-$Cl_2$Ph | Me | Me | 0 | 0 | 0 | 0 | 0 | 0 |
| 38 | 3,4-$Cl_2$Ph | Me | Et | 0 | 0 | 0 | 0 | 0 | 0 |
| 39 | p-MeOPh | Me | H | 0 | 0 | 0 | 0 | 0 | 0 |
| 40 | 3,4-$Cl_2$Ph | Me | H | 0 | 0 | 0 | 0 | 0 | 0 |

Table III

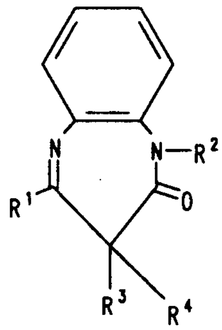

CONCENTRATION (PARTS PER MILLION) REQUIRED TO PROVIDE 50 PERCENT CONTROL

| COMPOUND | $R^1$ | $R^2$ | $R^3$ | Powdery Mildew |
|---|---|---|---|---|
| 8 | Ph | n-Bu | H | 40 |
| 9 | t-Bu | Bz | H | 35 |
| 12 | Ph | Me | Br | 19 |
| 15 | Ph | Me | Et | 4 |
| 16 | Ph | Me | n-Bu | 12 |
| 18 | t-Bu | Me | Et | 50 |
| 21 | Ph | Me | Me | 80 |
| 22 | Ph | Et | H | 60 |
| 23 | Ph | n-propyl | H | 70 |
| 24 | Ph | Et | Me | 32 |
| 25 | Ph | Et | Et | 30 |
| 26 | Ph | n-propyl | Me | 45 |
| 27 | 4-ClPh | Me | Me | 17 |
| 28 | 4-ClPh | Me | Et | 33 |

## Claims

**1.** A method of controlling fungi comprising contacting the fungi with a fungicidally effective amount of a benzodiazepine,

the benzodiazapine characterized by the structure:

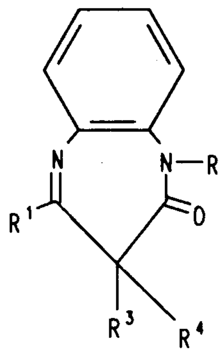

wherein

$R^1$ is substituted or unsubstituted alkyl having from 3 to 10 carbon atoms, or substituted or unsubstituted aryl having from 6 to 20 carbon atoms,

$R^2$ is substituted or unsubstituted alkyl having from 1 to 10 carbon atoms, or substituted or unsubstituted aryl having from 6 to 20 carbon atoms,

$R^3$ is hydrogen, halogen, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen,provided that when $R^4$ is halogen, $R^3$ is the same halogen.

**2.** The method of claim 1 wherein $R^1$ is tertiary butyl, phenyl, or 4-chlorophenyl.

**3.** The method as claimed in either of claims 1 or 2 wherein $R^2$ is methyl, ethyl, n-propyl, n-butyl, or benzyl.

**4.** The method as claimed in either of claims 2 or 3 wherein $R^3$ is hydrogen, allyl, bromo, chloro, n-butyl, ethyl, or methyl.

**5.** The method as claimed in either of claims 1 or 4 wherein $R^1$ is phenyl and $R^2$ is methyl.

**6.** The method of claim 1 wherein $R^1$ is phenyl, $R^2$ is methyl and $R^3$ is ethyl.

**7.** The method of claim 1 wherein $R^1$ is phenyl, $R^2$ is methyl and $R^3$ is n-butyl.

**8.** The method of claim 1 wherein $R^1$ is 4-chlorophenyl, $R^2$ is methyl and $R^3$ is methyl.

**9.** The method of claim 1 wherein $R^1$ is phenyl, $R^2$ is methyl and $R^3$ is bromo.

**10.** A substituted 1,5-benzodiazepine characterized as having the following structure:

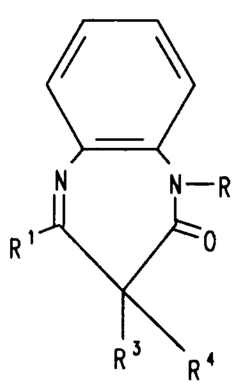

wherein

$R^1$ is substituted or unsubstituted alkyl having from 3 to 10 carbon atoms, or substituted or unsubstituted aryl having from 6 to 20 carbon atoms,

$R^2$ is substituted or unsubstituted alkyl having from 1 to 10 carbon atoms, or substituted or unsubstituted aryl having from 6 to 20 carbon atoms,

$R^3$ is hydrogen, bromo, chloro, iodo, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen, provided that when $R^4$ is halogen, $R^3$ is the same halogen, and when $R^1$ is methyl or phenyl and $R^2$ is allyl, ethyl, benzyl, propyl or butyl, then neither $R^3$ nor $R^4$ are hydrogen.

**11.** The compound of claim 10 wherein $R^1$ is tertiary butyl, phenyl, or 4-chlorophenyl.

**12.** The compound of either of claims 10 or 11 wherein $R^2$ is methyl, ethyl, n-propyl, n-butyl, or benzyl.

**13.** The compound of either of claims 10, 11 or 12 wherein $R^3$ is hydrogen, allyl, bromo, chloro, n-butyl, ethyl, or methyl.

**14.** The compound of claim 10 wherein $R^1$ is phenyl and $R^2$ is methyl.

**15.** The compound of claim 10 wherein $R^1$ is phenyl, $R^2$ is methyl and $R^3$ is ethyl.

**16.** The compound of claim 10 wherein $R^1$ is phenyl, $R^2$ is methyl and $R^3$ is n-butyl.

**17.** The compound of claim 10 wherein $R^1$ is 4-chlorophenyl, $R^2$ is methyl and $R^3$ is methyl.

**18.** The compound of claim 10 wherein $R^1$ is phenyl, $R^2$ is methyl and $R^3$ is bromo.

**19.** A fungicidal composition comprising a fungicidally effective amount of a benzodiazepine, the benzodiazepine characterized by the following structure:

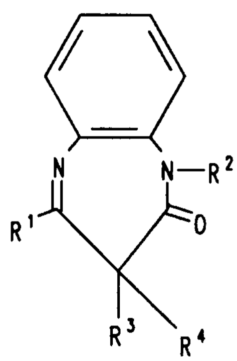

wherein

$R^1$ is substituted or unsubstituted alkyl having from 3 to 10 carbon atoms, or substituted or unsubstituted aryl having from 6 to 20 carbon atoms,

$R^2$ is substituted or unsubstituted alkyl having from 1 to 10 carbon atoms, or substituted or unsubstituted aryl having from 6 to 20 carbon atoms,

$R^3$ is hydrogen, halogen, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen, provided that when $R^4$ is halogen, $R^3$ is the same halogen.

**20.** A fungicidal composition comprising a fungicidally effective amount of at least one benzodiazepine, the benzodiazepine characterized as follows: 1-n-butyl-2,3-dihydro-2-oxo-4-phenyl-1H-1,5-benzodiazepine; 1-benzyl-4-t-butyl-2,3-dihydro-2-oxo-1H-1,5-benzodiazepine; 3-bromo-2,3-dihydro-1-methyl-2-oxo-4-phenyl-1H-1,5-benzodiazepine; 3-ethyl-2,3-dihydro-1-methyl-2-oxo-4-phenyl-1H-1,5-benzodiazepine; 3-n-butyl-2,3-dihydro-1-methyl-2-oxo-4-phenyl-1H-1,5-benzodiazepine; 4-t-butyl-2,3-dihydro-3-ethyl-1-methyl-2-oxo-1H-1,5-benzodiazepine; 2,3-dihydro-1,3-dimethyl-2-oxo-4-phenyl-1H-1,5-benzodiazepine; 2,3-dihydro-1-ethyl-2-oxo-4-phenyl-1H-1,5-benzodiazepine; 2,3-dihydro-2-oxo-4-phenyl-1-propyl-1H-1,5-benzodiazepine; 2,3-dihydro-1-ethyl-3-methyl-2-oxo-4-phenyl-1H-1,5-benzodiazepine; 1,3-diethyl-2,3-dihydro-2-oxo-4-phenyl-1H-1,5-benzodiazepine; 2,3-dihydro-3-methyl-2-oxo-4-phenyl-1-n-propyl-1H-1,5-benzodiazepine; 4-(4-chlorophenyl)-2,3-dihydro-1,3-dimethyl-2-oxo-1H-1,5-benzodiazepine; or 4-(4-chlorophenyl)-2,3-dihydro-3-ethyl-1-methyl-2-oxo-1H-1,5-benzo-diazepine.

**21.** The use of a substituted 1,5-benzodizaepine as a fungicide,
the benzodiazepine characterized as having the following structure:

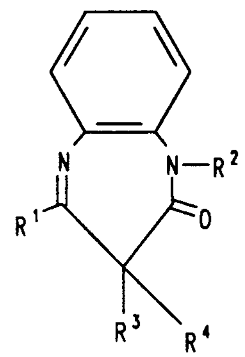

wherein

$R^1$ is substituted or unsubstituted alkyl having from 3 to 10 carbon atoms, or substituted or unsubstituted aryl having from 6 to 20 carbon atoms,

$R^2$ is substituted or unsubstituted alkyl having from 1 to 10 carbon atoms, or substituted or unsubstituted aryl having from 6 to 20 carbon atoms,

$R^3$ is hydrogen, halogen, substituted or unsubstituted alkyl having from 1 to 10 carbon atoms, alkenyl having from 2 to 10 carbon atoms, or substituted or unsubstituted aryl having from 6 to 20 carbon atoms, and

$R^4$ is hydrogen or halogen, provided that when $R^4$ is halogen, $R^3$ is the same halogen.

European Patent Office

# EUROPEAN SEARCH REPORT

Application Number

EP 91 20 2988
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,X | PESTICIDE SCIENCE<br>vol. 7, 1976, LONDON GB<br>pages 453 - 458;<br>D.P. CLIFFORD ET AL.: 'Herbicidal and Pesticidal Properties of Some 1,5-Benzodiazepines, 1,3,5-Benzotriazepines and 3,1,5-Benzothiadiazepines'<br>* the whole document *<br>--- | 1-9, 19-21 | C07D243/12<br>A01N43/62 |
| X | HETEROCYCLES<br>vol. 23, no. 4, 1985, SENDAI JP<br>pages 799 - 802;<br>E.M. ESSASSI ET AL.: 'Convenient Phase Transfer N-Arylation of 1,3-Dihydro-1,5-benzodiazepin-2-ones'<br>* the whole document, particularly page 800, compound IIb *<br>--- | 10-18 | |
| X | CHEMICA SCRIPTA<br>vol. 16, no. 5, 1980, STOCKHOLM SE<br>pages 157 - 162;<br>G. VERNIN ET AL.: 'Alkylation en Catalyse par Transfert de Phase de Dihydro-1,3(2H) Benzo (2,3b) Diazepines-1,5-ones-2. Anxiolytiques Potentiels'<br>* the whole document *<br>--- | 10-18 | TECHNICAL FIELDS SEARCHED (Int. Cl.5 )<br>C07D<br>A01N |
| X | IL FARMACO<br>vol. 44, no. 2, 1989, PAVIA IT<br>pages 125 - 140;<br>F. SAVELLI ET AL.: '1,3-Diidro-2H-1,5-benzo-e-clorobenzodiazepin-2-oni-1,4-disostiuiti ad attività sul S.N.C.'<br>* the whole document, particularly page 127, compounds (B) *<br>---<br>-/-- | 10-18 | |

The present search report has been drawn up for all claims

| Place of search | Date of completion of the search | Examiner |
|---|---|---|
| THE HAGUE | 26 FEBRUARY 1992 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 91 20 2988
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | BULLETIN DE LA SOCIéTé CHIMIQUE DE FRANCE no. 5, 1988, PARIS FR pages 889 - 896; R. ACHOUR ET AL.: 'Synthèse er réactivité de nouvelles molécules de type bis-(phényl-4 (cyclopropyl-4 et méthyl-4) dihydro-2,3 oxo-2 benzodiazépine-1,5 yl-1)-1,n alcanes' * the whole document, particularly page 890, compounds 6'a and 7'a * --- | 10-18 | |
| X | US-A-2 957 867 (L.H. WERNER) * the whole document * --- | 10-18 | |
| X | JP-A-62 174 062 (FUJISAWA PHARMACEUTICAL CO., LTD.) & CHEMICAL ABSTRACTS, vol. 108, no. 9, 29 February 1988, Columbus, Ohio, US; abstract no. 75434B, page 687 ; * abstract * --- | 10-18 | |
| | | | TECHNICAL FIELDS SEARCHED (Int. Cl.5 ) |
| A | EP-A-0 133 322 (DAIKIN INDUSTRIES, LIMITED) * the whole document * ----- | 1-21 | |

The present search report has been drawn up for all claims

| Place of search | Date of completion of the search | Examiner |
|---|---|---|
| THE HAGUE | 26 FEBRUARY 1992 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document